# EUROPÄISCHE PATENTSCHRIFT

(11) **EP 1 131 298 B1**
(45) Veröffentlichungstag und Bekanntmachung des Hinweises auf die Patenterteilung: **03.09.2003**
(21) Anmeldenummer: 99957286.0
(22) Anmeldetag: 06.11.1999
(51) Int. Cl.: C07D 231/20

(54) **VERFAHREN ZUR HERSTELLUNG VON 1-SUBSTITUIERTEN 5-HYDROXYPYRAZOLEN**
METHOD FOR THE PRODUCTION OF 1-SUBSTITUTED 5-HYDROXYPYRAZOLES
PROCEDE DE PREPARATION DE 5-HYDROXYPYRAZOLES SUBSTITUES EN POSITION 1

(30) Priorität: 19.11.1998 DE 19853501
(43) Veröffentlichungstag der Anmeldung: 12.09.2001
(73) Patentinhaber: BASF AKTIENGESELLSCHAFT, 67056 Ludwigshafen (DE)
(72) Erfinder: MAYWALD, Volker, D-67071 Ludwigshafen (DE); STEINMETZ, Adrian, D-68165 Mannheim (DE); RACK, Michael, D-69123 Heidelberg (DE); GÖTZ, Norbert, D-67547 Worms (DE); GÖTZ, Roland, D-68809 Neulussheim (DE); HENKELMANN, Jochem, D-68165 Mannheim (DE); BECKER, Heike, D-67227 Frankenthal (DE); AISCAR BAYETO, Juan Jose, D-68167 Mannheim (DE)
(86) Internationale Anmeldenummer: EP9908515
(87) Internationale Veröffentlichungsnummer: WO00031041

(56) Entgegenhaltungen:
- EP-A- 0 587 072
- EP-A- 0 837 058

## Beschreibung

Die vorliegende Erfindung betrifft ein Verfahren zur Herstellung von 1-substituierten 5-Hydroxy-pyrazolen der Formel I 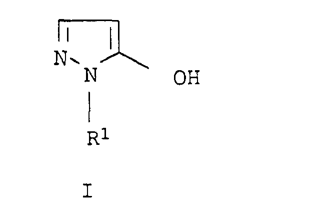 in der R¹ C₁-C₆-Alkyl, C₂-C₆-Alkenyl, C₂-C₆-Alkinyl, C₃-C₆-Cycloalkyl oder C₁-C₄-Alkoxy bedeutet, wobei diese Gruppen durch Halogen, C₁-C₄-Alkoxy, Phenoxy, C₁-C₆-Alkoxycarbonyl, C₁-C₆-Alkylthiocarbonyl oder ein cyclisches Ringsystem mit 3-14 Ringatomen substituiert sein können.

1-substituierte 5-Hydroxy-pyrazole finden als Vorprodukte zur Herstellung von Pharmaka und Pflanzenschutzmitteln, insbesondere Herbiziden, Verwendung und sind beispielsweise aus der WO 96/26206, WO 97/23135, WO 97/19087, US 5,631,210, WO 97/12885, WO 97/08164, ZA 9510980, WO 97/01550, WO 96/31507, WO 96/30368 und WO 96/25412 bekannt.

Verfahren zu deren Herstellung sind daher von Interesse.

Bisher sind als Verfahren zur Herstellung von niederen 1-Alkyl-5-hydroxy-pyrazolen folgende Synthesen bekannt:
1. eine Darstellung, bei der 2-Methyl-1-(p-toluolsulfonyl)-3-pyrazolidon oder 2-Methyl-1-acetyl-pyrazolidon hydrolysiert wird (J. Prakt. Chem. 1971, 313, 115-128 und J. Prakt. Chem. 1971, 313, 1118-1124).
2. ein Variante, bei der 5-Hydroxy-1-alkylpyrazol-4-carbonsäurealkylester durch Cyclisierung eines Alkoxymethylenmalonsäuredialkylesters mit niederen Alkylhydrazinen aufgebaut wird, anschließend zu diesem Reaktionsprodukt eine wäßrige Mineralsäure-Lösung zugesetzt, und die Hydrolyse und Decarboxylierung gleichzeitig durchgeführt wird (siehe JP 61257974, JP 60051175, JP 58174369, J? 58140073 und JP 58140074 sowie US 4643757).
3. eine Synthese, bei der Propiolsäureethylester mit Methylhydrazin zu 5-Hydroxy-1-methyl-pyrazol umgesetzt wird (Annalen 1965, 686, 134-144).
4. eine Syntheseroute, bei der 3-Hydrazinopropionsäureester, die durch Addition von Hydrazin an Acrylsäureester entstehen, mit Aldehyden zu den entsprechenden Hydrazonen umgesetzt und anschließend cyclisiert werden (siehe JP 06166666, JP 61229852 und JP 61268659 sowie EP 240001).
5. ein Synthesevariante, bei der eine 5-Hydroxy-1-methylpyrazol-3-carbonsäure thermisch gespalten wird (Chem. Ber. 1976, 109, S. 261).
6. ein Verfahren, bei dem 3-Alkoxyacrylsäureester mit Methylhydrazin bzw. Ethylhydrazin zu 1-Methyl-5-hydroxy-pyrazol bzw. 1-Ethyl-5-hydroxy-pyrazol umgesetzt werden (siehe JP 189 271/86, EP-A-837 058).

Bei dem oben genannten 1. Syntheseweg ist der Verfahrensprozeß mehrstufig und kompliziert. Das Einfügen und Entfernen einer Schutzgruppe ist umständlich, bedeutet zusätzliche Stufenzahlen und vermindert die Ausbeute.

Die 2. Darstellungsmöglichkeit ist mehrstufig, außerdem entstehen neben den 1-Alkyl-5-hydroxy-pyrazolen gleichzeitig die Regioisomeren der Zielverbindung, die aufwendig von den Zielverbindungen abgetrennt werden müssen. Zudem ist die Synthese mit einer schlechten C-Ausbeute verbunden, da ein C4-Baustein eingesetzt wird, wovon am Ende des Verfahrens wieder ein C-Atom abgespalten werden muß.

Bei der 3. Synthesevariante, die lediglich die Darstellung von 1-Methyl-5-hydroxy-pyrazol beschreibt, ist es unabdingbar, weit überstöchiometrische Mengen an Methylhydrazin einzusetzen, wodurch das Verfahren unwirtschaftlich ist. Daneben muß das ebenfalls entstehende Isomer 3-Hydroxy-1-methylpyrazol bei der Reinigung aufwendig von 1-Methyl-5-hydroxy-pyrazol getrennt werden. Des weiteren ist dieses Verfahren aufgrund des hohen Preises von Propiolsäureester unwirtschaftlich.

Bei der 4. Alternative ist der Verfahrensprozeß mehrstufig und kompliziert. Die letzte Stufe des aufwendigen Verfahrens liefert nur schlechte Ausbeuten und eine Vielzahl von Nebenprodukten.

Bei dem 5. Synthesezugang ist zur thermischen Abspaltung eine hohe Temperatur erforderlich, die Ausbeute ist mit 6% sehr gering.

Beim 6. Syntheseweg, der lediglich die Darstellung von 1-Methyl-5-hydroxy-pyrazol beschreibt, werden aufwendig darzustellende und teure 3-Alkoxyacrylsäureester eingesetzt. Die Herstellung von 3-Alkoxyacrylsäureester erfolgt durch Umsetzung von Methanol mit teuren Propiolsäurestern (Tetrahedron Lett. 1983, 24, 5209, J. Org. Chem. 1980, 45, 48, Chem. Ber. 1966, 99, 450, Chem. Lett. 1996, 9, 727-728), durch Umsetzung teurer und aufwendig zu synthetisierenden α,α-Dichlor-diethylether mit Bromessigsäureestern (Zh. Org. Khim. 1986, 22, 738), durch Umsetzung von Bromessigsäureestern mit Trialkylformiaten (Bull. Soc. Chim. France 1983, N 1-2, 41-45) und durch Abspaltung von Methanol aus 3,3-Dialkoxypropionsäureestern (DE 3701113) (erhältlich durch Umsetzung des teuren Propiolsäuremethylester mit Methanol (J. Org. Chem. 1976, 41, 3765), durch Umsetzung von 3-N-Acetyl-N-alkyl-3-methoxy-propionsäureestern mit Methanol (J. Org. Chem. 1985, 50, 4157-4160, JP 60-156643), durch Umsetzung von Acrylsäureestern mit Alkylaminen und Essigsäureanhydrid (J. Org. Chem. 1985, 50, 4157-4160), durch Umsetzung von Keten mit Trialkylorthoformiat (DK 158462), durch Palladium-und gleichzeitiger Kupfer-katalysierte Reaktion von Acrylsäureestern mit Methanol (DE 4100178.8), durch Umsetzung von Trichloracetylchlorid mit Vinylethylether (Synthesis 1988, 4, 274), durch Umsetzung von α,α,α-Trichlor-ß-methoxy-buten-2-on mit Methanol (Synthesis 1988,4, 274) und durch Umsetzung der Natriumsalze von 3-Hydroxyacrylsäureestern mit Alkoholen (DB 3641605)) eingesetzt werden. Die schlechte Zugänglichkeit der 3-Alkoxyacrylsäureester macht die Synthese nach 6. daher unwirtschaftlich. Nach JP 189 271/86 wird außerdem lediglich die Isolierung des 5-Hydroxy-1-methylpyrazols als Hydrochlorid beschrieben, jedoch keine Aussage zur Isolierung und Reinigung der freien Base beschrieben. Versucht man die in JP 189 271/86 beschriebenen Reaktionsbedingungen anzuwenden und die freie Base zu isolieren, erhält man nur sehr geringe Ausbeuten, die für eine großtechnische Herstellung von Hydroxypyrazolen unwirtschaftlich sind. Aus der EP-A 837 058 ist lediglich die Herstellung des 5-Hydroxy-1-ethyl-pyrazols bekannt.

Folglich können diese Syntheserouten als wirtschaftliche und effiziente Verfahren zur Herstellung von 1-substituierten-5-Hydroxy-pyrazolen nicht zufriedenstellen. Dies trifft insbesondere zu für den Fall, der technischen Herstellung der 1-substituierten-5-Hydroxy-pyrazole in großen Mengen.

Aufgabe der vorliegenden Erfindung war es daher, ein alternatives Herstellungsverfahren zur Herstellung von 1-substituierten-5-Hydroxy-pyrazolen zu finden, das die oben genannten Nachteile der bisher bekannten Herstellungsmethoden nicht aufweist.

Diese Aufgabe wurde überraschenderweise gelöst durch das erfindungsgemäße Verfahren zur Herstellung von 1-substituierten 5-Hydroxypyrazolen der Formel I 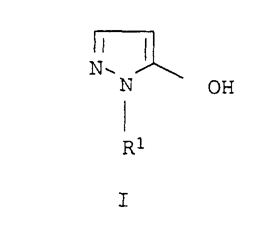 in der R¹ C₁-C₆-Alkyl, C₂-C₆-Alkenyl, C₂-C₆-Alkinyl, C₃-C₆-Cycloalkyl oder C₁-C₄-Alkoxy bedeutet, wobei diese Gruppen durch Halogen, C₁-C₄-Alkoxy, Phenoxy, C₁-C₆-Alkoxycarbonyl, C₁-C₆-Alkylthiocarbonyl oder ein cyclisches Ringsystem mit 3-14 Ringatomen substituiert sein können, indem man
a) einen Alkylvinylether der allgemeinen Formel III, 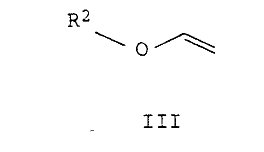 in der R² C₁-C₆-Alkyl oder C₃-C₆-Cycloalkyl bedeutet, mit Phosgen IVa, "Diphosgen" IVb oder "Triphosgen" IVc 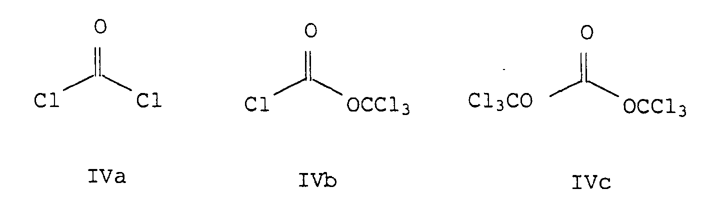 zu Säurechloriden der Formel V 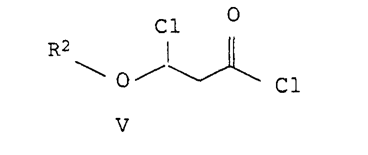 umsetzt,
b) diese durch Eliminierung von Chlorwasserstoff in das entsprechende 3-Alkoxyacrylsäurechlorid der Formel VI 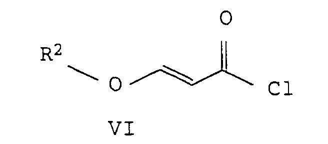 überführt und
c) dieses mit Hydrazinen der Formel VII 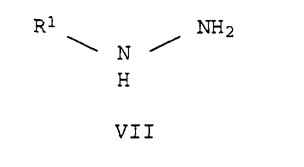 in der R¹ die oben angegebene Bedeutung hat, zu 5-Hydroxypyrazolen der Formel I umsetzt.

Bevorzugte Ausführungsformen des erfindungsgemäßen Verfahrens sind den Unteransprüchen und der nachfolgenden Beschreibung zu entnehmen.

### Stufe a):

Ausgangspunkt des erfindungsgemäßen Verfahrens sind Alkylvinylether der Formel III, die zunächst bei Temperaturen von -78°C bis 100°C, vorzugsweise -10°C bis 80°C, insbesondere 20°C bis 60°C, mit einem Säurechlorid der Formel IVa, IVb oder IVc umgesetzt werden, wobei sich das entsprechende Säurechlorid der Formel V bildet.

Die Umsetzung kann ohne Verwendung von Lösungs- bzw. Verdünnungsmitteln durchgeführt werden, sofern die Umsetzungspartner bei der Umsetzungstemperatur flüssig sind. Es ist aber auch möglich, die Umsetzung in einem aprotischen Lösungs- oder Verdünnungsmittel durchzuführen.

Geeignete Lösungs- oder Verdünnungsmittel sind beispielsweise aliphatische Kohlenwasserstoffe wie Pentan, Hexan, Cyclohexan und Petrolether, aromatische Kohlenwasserstoffe wie Toluol, o-, mund p-Xylol, halogenierte Kohlenwasserstoffe wie Methylenchlorid, Chloroform und Chlorbenzol, sowie Ether wie Diethylether, Diisopropylether, tert.-Butylmethylether, Dioxan, Anisol und Tetrahydrofuran, und Nitrile wie Acetonitril und Propionitril. Natürlich können auch Gemische der genannten Lösungsmittel verwendet werden.

Besonders bevorzugt führt man die Umsetzung ohne Lösungsmittel oder in aromatischen Kohlenwasserstoffen wie Toluol als Lösungsmittel durch.

Die Umsetzungspartner III und IV werden im allgemeinen im Verhältnis von 0,1:1 bis 1:1 mol III/IVa, IVb bzw. IVc, vorzugsweise 0,2:1 bis 0,8:1 mol III/IVa, IVb bzw. IVc, insbesondere 0,4:1 bis 0,6:1 mol III/IVa, IVb bzw. IVc, miteinander umgesetzt.

Da sowohl die Halogenide IV als auch das gebildete Säurechlorid V gegen Feuchtigkeit labil sind, empfiehlt es sich, die Umsetzung unter Ausschluß von Wasser, vorzugsweise unter Schutzgasatmosphäre (Stickstoff oder ein anderes Inertgas) durchzuführen.

Im Fall der Umsetzung von III mit IVb bzw. IVc kann es von Vorteil sein, die Reaktion durch die Zugabe von katalytischen Mengen eines tertiären Amins wie Triethylamin und Pyridin zu beschleunigen.

### Stufe b):

Das so gewonnene Säurechlorid V spaltet bei 30 bis 80°C Chlorwasserstoff (HCl) ab, wobei sich das entsprechende 3-Alkoxyacrylsäurechlorid VI bildet. Die Herstellung dieses Säurechlorides VI ist in der EP-A 0 587 072 beschrieben.

Für diese Stufe der Umsetzung kann es von Vorteil sein, den gebildeten Chlorwasserstoff aus dem Reaktionsvolumen zu entfernen, sei es durch leichten Unterdruck oder dadurch, daß man Inertgas durch die Reaktionsmischung oder das Reaktionsgefäß leitet und somit den gebildeten Chlorwasserstoff entfernt.

Das überschüssige Chlorid der Formel IVa, IVb bzw. IVc kann in die Synthese rückgeführt werden und muß in jedem Fall zur Isolierung des reinen Wertproduktes abgetrennt werden. Gleiches gilt für evtl. zugesetzte Katalysatoren.

Die so erhaltenen rohen 3-Alkoxyacrylsäurechloride VI können durch Destillation oder Rektifikation in reiner Form isoliert werden.

Sie können aber auch direkt, ohne weitere Reinigung weiterverwendet werden.

### Stufe c):

Bei der Umsetzung der 3-Alkoxyacrylsäurechloride der Formel VI mit Hydrazinen der Formel VII zu den 1-substituierten 5-Hydroxypyrazolen wird im allgemeinen so vorgegangen, daß man das Hydrazin VII bei Temperaturen von -20 bis 80°C, bevorzugt -20°C bis 50°C, in einem organischen Lösungsmittel vorlegt und das Säurechlorid VI innerhalb von 0,2 - 3 h zutropft.

Geeignete Lösungs- oder Verdünnungsmittel sind beispielsweise aliphatische Kohlenwasserstoffe wie Pentan, Hexan, Cyclohexan und Petrolether, aromatische Kohlenwasserstoffe wie Toluol, o-, mund p-Xylol, halogenierte Kohlenwasserstoffe wie Methylenchlorid, Chloroform und Chlorbenzol, sowie Ether wie Diethylether, Diisopropylether, tert.-Butylmethylether, Dioxan, Anisol und Tetrahydrofuran, und Nitrile wie Acetonitril und Propionitril. Natürlich können auch Gemische der genannten Lösungsmittel verwendet werden.

Zur Cyclisierung des gebildeten Hydrazids gibt man eine organische oder anorganische Säure zu und erhitzt die Reaktionsmischung auf Temperaturen von 30 - 100°C.

Geeignete organische oder -anorganische Säuren sind Trifluormethansulfonsäure, p-Toluolsulfonsäure, Schwefelsäure, Salzsäure, Phosphorsäure. Besonders bevorzugt werden Mineralsäuren wie Schwefelsäure und Salzsäure verwendet.

Im Hinblick auf die bestimmungsgemäße Verwendung der 1-substituierten 5-Hydroxypyrazolen der allgemeinen Formel I kommen als Substituenten folgende Reste in Betracht:
R¹
C₁-C₄-Alkyl, wie Methyl, Ethyl, n-Propyl, 1-Methylethyl, Butyl, 1-Methylpropyl, 2-Methylpropyl und 1,1-Dimethylethyl;
C₁-C₆-Alkyl, wie C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
insbesondere Methyl, Ethyl, 1-Methylethyl, 1-Methylpropyl, 2-Methylpropyl, 1,1-Dimethylethyl und 1,1-Dimethylpropyl;
C₂-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-4-butenyl, 3-Methyl-3 butenyl, 1,1-Dimethyl-2-propenyl,1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3 pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und Ethyl-2-methyl-2-propenyl,
insbesondere 1-Methyl-2-propenyl, 1-Methyl-2-butenyl, 1,1-Dimethyl-2-propenyl und 1,1-Dimethyl-2-butenyl;
C₂-C₆-Alkinyl wie Propargyl, 2-Butinyl, 3-Butinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-l-methyl-2-propinyl;
C₃-C₆-Cycloalkyl, wie beispielsweise Cyclopropyl, Cyclobutyl, Cyclopentyl und Cyclohexyl,
insbesondere Cyclopropyl und Cyclohexyl;
C₁-C₄-Alkoxy wie Methoxy, Ethoxy, n-Propoxy, 1-Methylethoxy, n-Butoxy, 1-Methylpropoxy, 2-Methylpropoxy und 1,1-Dimethylethoxy,
insbesondere C₁-C₃-Alkoxy wie Methoxy, Ethoxy, i-Propoxy;
wobei diese Gruppen gegebenenfalls durch ein bis fünf Halogenatome wie Fluor, Chlor, Brom und Iod, vorzugsweise Fluor und Chlor, C₁-C₄-Alkoxy, Phenoxy, C₁-C₆-Alkoxycarbonyl, C₁-C₆-Alkylthiocarbonyl oder ein cyclisches Ringsystem mit 3-14 Ringatomen substituiert sein können, wobei die Substituenten die folgende Bedeutung haben:
C₁-C₆-Alkoxycarbonyl wie Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl und 1,1-Dimethylethoxycarbonyl, insbesondere Methoxycarbonyl;
C₁-C₆-Alkylthiocarbonyl wie Methylthiocarbonyl, Ethylthiocarbonyl n-Propylthiocarbonyl, insbesondere Methylthiocarbonyl.
C₁-C₄-Halogenalkyl: einen C₁-C₄-Alkylrest wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, l-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;

Ein cyclisches Ringsystem mit 3 - 14 Ringatomen bedeutet beispielsweise die folgenden Gruppen: C₃-C₁₄-Cycloalkyl, C₃-C₁₄-Cycloalkenyl, aromatische Gruppen, wie Phenyl, Naphthyl, sowie deren teilhydrierten Derivate. Ferner können die cyclischen Ringsysteme heterocyclische Ringsysteme darstellen, in denen ein, zwei oder drei Kohlenstoffatome durch Heteroatome, wie z.B. O, N, S, ersetzt sein können. Grundsätzlich können die cyclischen Ringsysteme aromatisch, teilhydriert oder vollständig hydriert sein. Die cyclischen Ringsysteme können beliebig substituiert sein. Als Substituenten geeignet sind beispielsweise C₁-C₆-Alkyl, C₁-C₄-Halogenalkyl, C₁-C₄-Alkoxy, Halogen, Cyano, Nitro, Hydroxyl, Thionyl, Sulfoxyl, Sulfonyl, C₁-C₄-Alkylsulfonyl, Amino, C₁-C₄-Alkylamino und Di-C₁-C₄-Alkylamino.

Bevorzugt sind cyclische Ringsysteme aus der Gruppe C₁-C₆-Cycloalkyl, Phenyl, ein 5 bis 6-gliedriger heterocyclischer, gesättigter oder ungesättigter Rest, enthaltend ein bis drei Heteroatome, ausgewählt aus der Grupppe O, N und S, die wie oben angegeben substituiert sein können.

Besonders bevorzugt ist C₁-C₆-Cycloalkyl und Phenyl, die wie oben angegeben substituiert sein können.

Ein ganz besonders bevorzugtes cyclisches Ringsystem ist Phenyl, das wie oben angegeben substituiert sein kann.

R², R³ unabhängig voneinander C₁-C₆-Alkyl wie oben genannt oder C₃-C₆-Cycloalkyl, bevorzugt C₁-C₆-Alkyl.

### Beispiele

### Beispiel 1

### 3-Ethoxyacrylsäurechlorid

Zu einer Lösung aus 72 g (1mol) Ethylvinylether in 100 g Toluol werden 110 g (1,1 mol) Phosgen bei 35°C innerhalb von 1,5 h eingeleitet. Anschließend wird 4 Stunden bei 60°C nachgerührt. Während der gesamten Versuchszeit werden Phosgen und Ethylvinylether mit einem -78°C-Trockeneiskühler in die Reaktionsmischung rückkondensiert. Anschließend wird die Lösung bei Raumtemperatur phosgenfrei gestrippt und das Lösungsmittel destillativ entfernt. Nach Vakuumdestillation bei 36°C/0,4 mbar werden 88 g (66 %) Wertprodukt erhalten.

### Beispiel 2

### Isobutoxyacrylsäurechlorid

100 g (1 mol) Isobutylvinylether werden in einer 2 1 Rührapparatur vorgelegt und der Inhalt wird auf 50-55°C erwärmt. Anschließend werden 1024 g (10,4 mol) Phosgen innerhalb von 21 h eingeleitet und 900 g (9 mol) Isobutylvinylether innerhalb von 19 h zugetropft. Nach einer Nachreaktionszeit von 0,5 Stunden wird der Ansatz unter Stickstoffstrippung auf 80°C erwärmt, um Chlorwasserstoff abzuspalten. Danach werden die Niedersieder über eine 15 cm Vigreux-Kolonne abdestilliert und der Rückstand gaschromatographisch analysiert. Es werden 1364 g (70%) rohes Isobutoxyacrylsäurechlorid (ber. 100%) erhalten.

### Beispiel 3

### 3-Cyclohexyloxyacrylsäurechlorid

In eine mit -78°C-Kühlung ausgestattete Rührapparatur werden 50 g (0,5 mol) Phosgen einkondensiert. Anschließend werden innerhalb von 3 Stunden 50,5 g (0,4 mol) Cyclohexylvinylether bei 20°C eingetropft. Danach wird 5 Stunden bei 50°C nachgerührt. Das überschüssige Phosgen wird mit Stickstoff ausgetrieben und das Rohprodukt destillativ aufgearbeitet. Bei 110°C/2,5mbar wurden 66,4g (88 %) Wertprodukt erhalten.

### Beispiel 4

### 5-Hydroxy-1-methylpyrazol aus 3-Isobutoxyacrylsäurechlorid und Monomethylhydrazin (1,8 %ig)

In einem 6 1 Kolben werden 2377g einer wäßrigen Monomethylhydrazin-Lösung (0,93 mol) bei 10°C vorgelegt. Durch Zugabe von 38%iger Salzsäure wird ein pH-Wert von 7 eingestellt. Anschließend werden 1900 ml THF und 94,1 g (0,93 mol) Triethylamin zugegeben, wodurch der pH-Wert auf einen Wert von 10 ansteigt. Anschließend werden bei 10°C innerhalb von 7 min 60 g (0,365 mol) 3-Isobutoxyacrylsäurechlorid zugetropft. Nachdem 28 min bei gleicher Temperatur gerührt wird, erfolgt die Zugabe von weiteren 17 g (0,103 mol) 3-Isobutoxyacrylsäurechlorid innerhalb von 29 min. Zur Aufarbeitung werden die Phasen getrennt und die untere Phase mit 2 1 THF extrahiert. Die gesammelten organischen Phasen werden vereinigt und das Lösungsmittel im Vakuum abdestilliert. Es werden 78,3 g Zwischenprodukt erhalten, das in 625 g 10%iger Schwefelsäure gelöst wird. Die Reaktionsmischung wird für 70 min auf 90°C erhitzt. Gaschromatographische Analyse des Reaktionsgemisches ergab eine Ausbeute von 64 % (bezogen auf 3-Isobutoxyacrylsäurechlorid). Isomerenverhältnis: ≥ 100 : 1

Nach den oben beschriebenen Verfahren wurden auf analoge Weise folgende Verbindungen hergestellt.

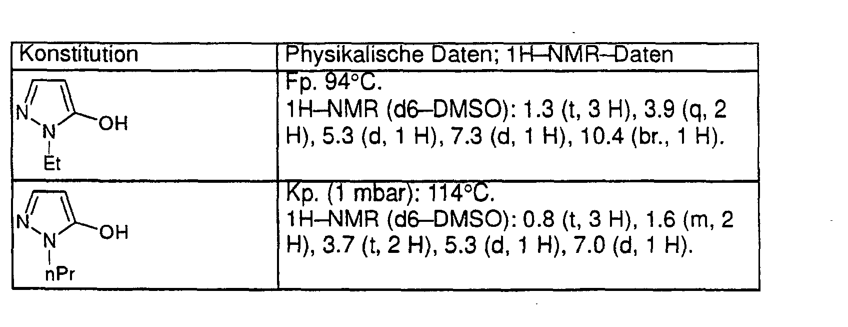

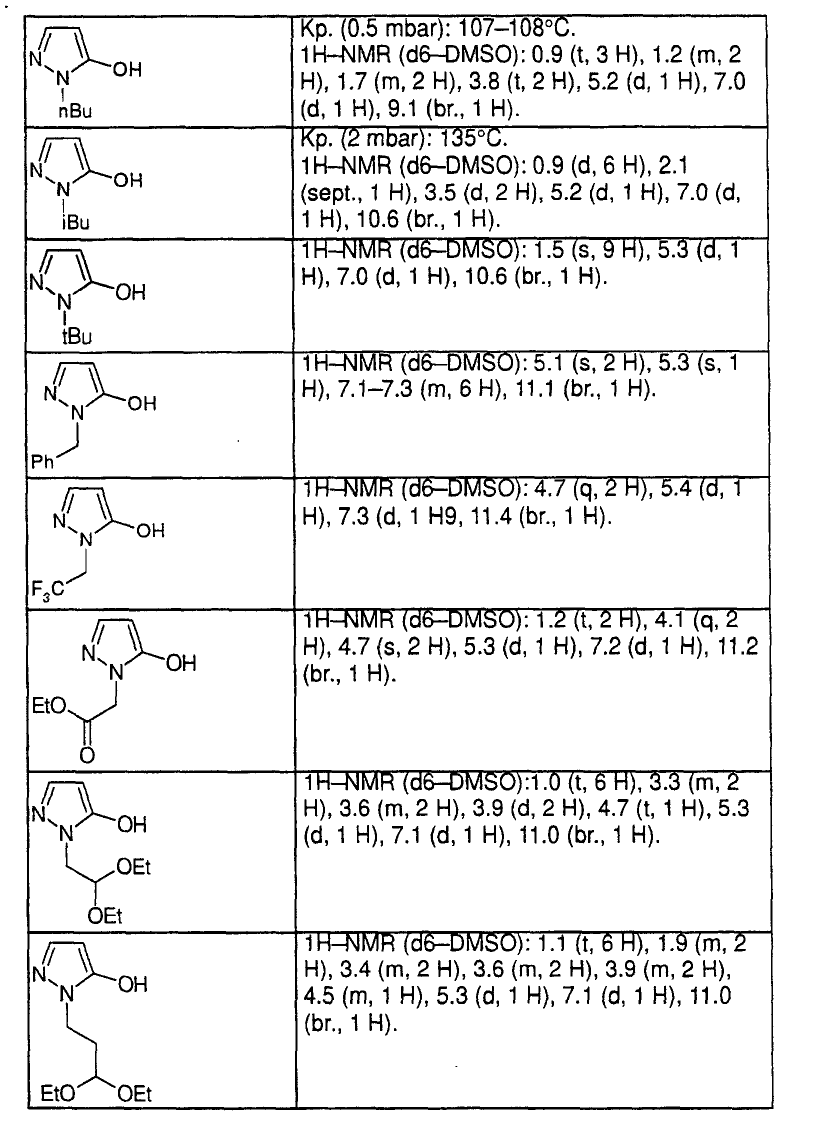

Die nach dem erfindungsgemäßen Verfahren hergestellten 1-substituierten 5-Hydroxypyrazole sind wertvolle Vorprodukte zur Herstellung von beispielsweise Pflanzenschutzmitteln wie Herbiziden. Aus der WO 96/26206 bekannte Herbizide sind beispielsweise

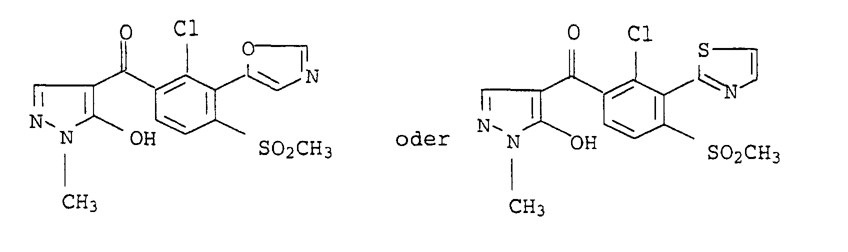

## Patentansprüche

1. Verfahren zur Herstellung von 1-substituierten 5-Hydroxypyrazolen der Formel I 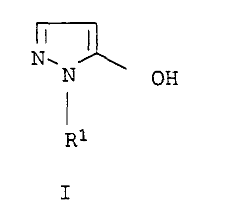 in der R¹ C₁-C₆-Alkyl, C₂-C₆-Alkenyl, C₂-C₆-Alkinyl, C₃-C₆-Cycloalkyl oder C₁-C₄-Alkoxy bedeutet, wobei diese Gruppen durch Halogen, C₁-C₄-Alkoxy, Phenoxy, C₁-C₆-Alkoxycarbonyl, C₁-C₆-Alkylthiocarbonyl oder ein cyclisches Ringsystem mit 3-14 Ringatomen substituiert sein können, **dadurch gekennzeichnet, daß** man
a) einen Alkylvinylether der allgemeinen Formel III, 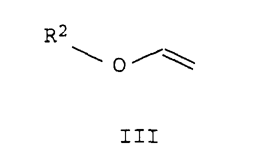 in der R² C₁-C₆-Alkyl oder C₃-C₆-Cycloalkyl bedeutet, mit Phosgen IVa, "Diphosgen" IVb oder "Triphosgen" IVc 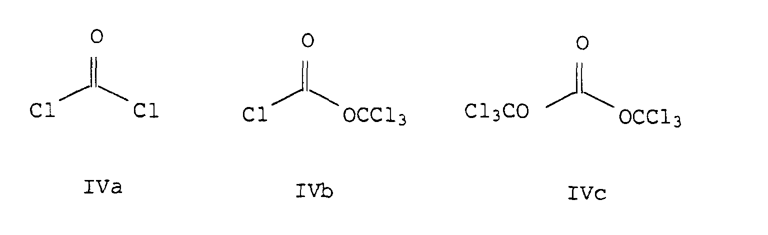 zu Säurechloriden der Formel V 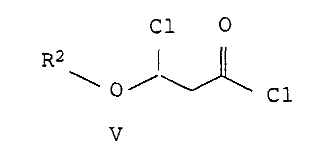 umsetzt,
b) diese durch Eliminierung von Chlorwasserstoff in das entsprechende 3-Alkoxyacrylsäurechlorid der Formel VI 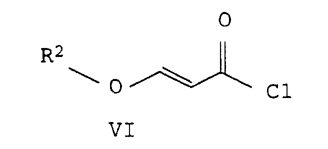 überführt und
c) dieses mit Hydrazinen der Formel VII 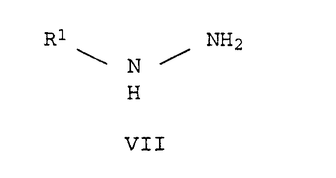 in der R¹ die oben angegebene Bedeutung hat, zu 5-Hydroxypyrazolen der Formel I umsetzt.

2. Verfahren nach Anspruch 1, **dadurch gekennzeichnet, daß** man die Umsetzung in Stufe a) bei einer Temperatur von -78°C bis 100°C durchführt.

3. Verfahren nach Anspruch 1, **dadurch gekennzeichnet, daß** man den Alkylvinylether III mit Phosgen IVa, Diphosgen IVb oder Triphosgen IVc im Molverhältnis 0,1:1 bis 1:1 umsetzt.

4. Verfahren nach Anspruch 1, **dadurch gekennzeichnet, daß** man die Umsetzung in Stufe b) bei einer Temperatur von 30°C bis 80°C durchführt.

5. Verfahren nach Anspruch 1, **dadurch gekennzeichnet, daß** man die Umsetzung in Stufe c) bei einer Temperatur von -20°C bis 80°C durchführt.

6. Verfahren nach Anspruch 1, **dadurch gekennzeichnet, daß** man die 3-Alkoxyacrylsäurechloride VI mit Hydrazinen VII bei einer Temperatur von -20°C bis 50°C zu einem Hydrazid umsetzt und dieses anschließend in Gegenwart einer organischen oder anorganischen Säure bei Temperaturen von 30 bis 100°C cyclisiert.

7. Verfahren nach Anspruch 6, **dadurch gekennzeichnet, daß** man als anorganische Säure Schwefelsäure, Salzsäure oder Phosphorsäure verwendet.

## Claims

1. A process for preparing a 1-substituted 5-hydroxypyrazole of the formula I 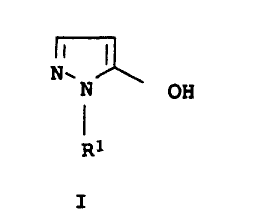 in which R¹ is C₁-C₆-alkyl, C₂-C₆-alkenyl, C₂-C₆-alkynyl, C₃-C₆-cycloalkyl or C₁-C₄-alkoxy, where these groups may be substituted by halogen, C₁-C₄-alkoxy, phenoxy, C₁-C₆-alkoxycarbonyl, C₁-C₆-alkylthiocarbonyl or by a cyclic ring system having 3-14 ring atoms, which comprises
a) reacting an alkyl vinyl ether of the formula III 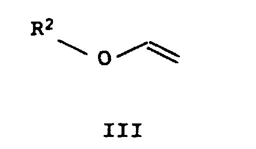 in which R² is C₁-C₆-alkyl or C₃-C₆-cycloalkyl with phosgene IVa, "diphosgene" IVb or "triphosgene" IVc 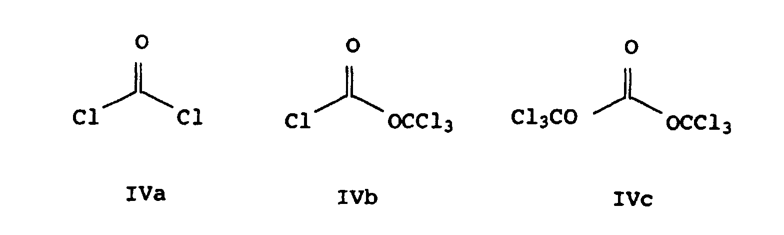 to give an acyl chloride of the formula V 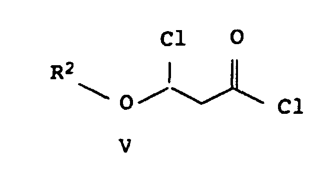
b) converting this by elimination of hydrogen chloride into the corresponding 3-alkoxyacryloyl chloride of the formula VI 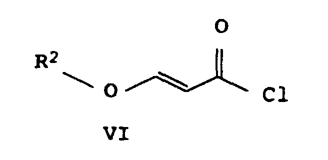 and
c) reacting this with a hydrazine of the formula VII 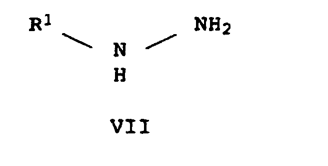 in which R¹ is as defined above to give a 5-hydroxypyrazole of the formula I.

2. A process as claimed in claim 1, wherein the reaction in step a) is carried out at from -78°C to 100°C.

3. A process as claimed in claim 1, wherein the alkyl vinyl ether III is reacted with phosgene IVa, diphosgene IVb or triphosgene IVc in a molar ratio of from 0.1:1 to 1:1.

4. A process as claimed in claim 1, wherein the reaction in step b) is carried out at from 30°C to 80°C.

5. A process as claimed in claim 1, wherein the reaction in step c) is carried out at from -20°C to 80°C.

6. A process as claimed in claim 1, wherein the 3-alkoxyacryloyl chloride VI is reacted with a hydrazine VII at from -20°C to 50°C to give a hydrazide which is subsequently cyclized in the presence of an organic or inorganic acid at from 30 to 100°C.

7. A process as claimed in claim 6, wherein the inorganic acid used is sulfuric acid, hydrochloric acid or phosphoric acid.

## Revendications

1. Procédé pour la préparation de 5-hydroxypyrazoles substitués en position 1 répondant à la formule I 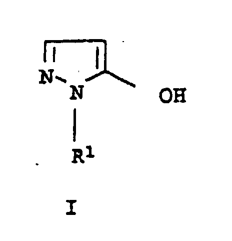 dans laquelle R¹ représente un groupe alkyle en C₁ - C₆, un groupe alcényle en C₂ - C₆, un groupe alcynyle en C₂ - C₆, un groupe cycloalkyle en C₃ - C₆ ou un groupe alcoxy en C₁ - C₄, ces groupes pouvant porter un ou plusieurs substituants comprenant un atome d'halogène, un groupe alcoxy en C₁ - C₄, un groupe phénoxy, un groupe alcoxy(en C₁ - C₆)carbonyle, un groupe alcoxy(en C₁ - C₆)thiocarbonyle ou un système nucléaire cyclique contenant de 3 à 14 atomes nucléaires, **caractérisé en ce qu'**on fait réagir
a) un éther alkylvinylique répondant à la formule générale III 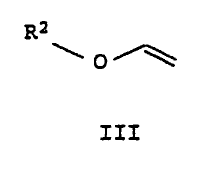 dans laquelle R² représente un groupe alkyle en C₁ - C₆ ou un groupe cycloalkyle en C₃ - C₆, avec du phosgène IVa, du "diphosgène" IVb ou du "triphosgène" IVc 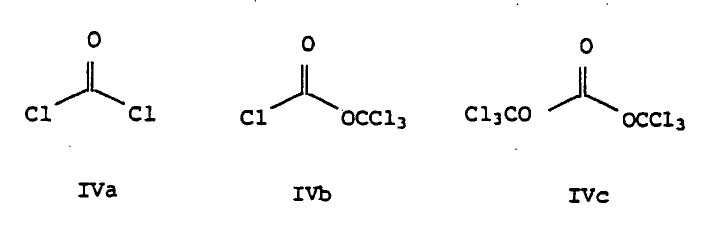 pour obtenir des chlorures d'acides répondant à la formule V 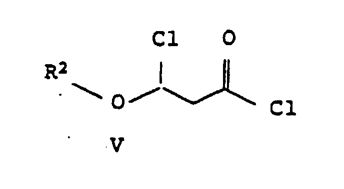
b) on transforme ces derniers, par élimination de l'acide chlorhydrique, en chlorure d'acide 3-alcoxyacrylique correspondant répondant à la formule VI 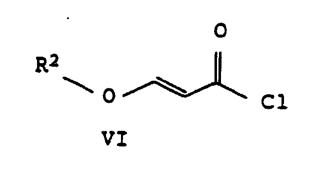 et,
c) on fait réagir ce dernier avec des hydrazines répondant à la formule VII 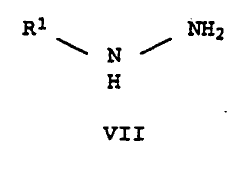
dans laquelle R¹ a la signification indiquée ci-dessus, pour obtenir des 5-hydroxypyrazoles répondant à la formule I.

2. Procédé selon la revendication 1, **caractérisé en ce qu'**on effectue la mise en réaction à l'étape a) à une température de -78 °C à 100 °C.

3. Procédé selon la revendication 1, **caractérisé en ce qu'**on fait réagir l'éther alkylvinylique III avec le phosgène IVa, le diphosgène IVb ou le triphosgène IVc dans le rapport molaire de 0,1 : 1 à 1 : 1.

4. Procédé selon la revendication 1, **caractérisé en ce qu'**on effectue la mise en réaction à l'étape b) à une température de 30 °C à 80 °C.

5. Procédé selon la revendication 1, **caractérisé en ce qu'**on effectue la mise en réaction à l'étape c) à une température de -20 °C à 80 °C.

6. Procédé selon la revendication 1, **caractérisé en ce qu'**on fait réagir les chlorures d'acide 3-alcoxyacrylique VI avec des hydrazines VII à une température de -20 °C à 50 °C pour obtenir un hydrazide et on soumet ensuite ce dernier à une cyclisation en présence d'un acide organique ou inorganique à des températures de 30 à 100 °C.

7. Procédé selon revendication 6, **caractérisé en ce qu'**on utilise, à titre d'acides inorganiques, de l'acide sulfurique, de l'acide chlorhydrique ou de l'acide phosphorique.
